# EUROPEAN PATENT SPECIFICATION

(11) **EP 4 023 151 B1**
(45) Date of publication and mention of the grant of the patent: **15.05.2024**
(21) Application number: 21217710.9
(22) Date of filing: 24.12.2021
(51) Int. Cl.: A61B 5/0538, A61B 18/00, A61B 34/20, A61B 18/14, A61B 5/00

(54) **DETECTING ELECTRODE WIRE NOISE**
DETEKTION VON ELEKTRODENDRAHTRAUSCHEN
DÉTECTION DE BRUIT DE FIL D'ÉLECTRODE

(30) Priority: 29.12.2020 US 202017136596
(43) Date of publication of application: 06.07.2022
(73) Proprietor: Biosense Webster (Israel) Ltd, Yokneam, 2066717 (IL)
(72) Inventor: BOTZER, Lior, 2066717 Yokneam (IL); BAR-TAL, Meir, 2066717 Yokneam (IL)
(74) Representative: Carpmaels & Ransford LLP

(56) References cited:
- US-A1- 2010 076 454
- US-A1- 2011 152 712
- US-A1- 2020 170 525

## Description

### FIELD OF INVENTION

The present application is directed to detection of electrode wire noise.

### BACKGROUND

Probes, such as catheters, are used in a wide range of applications, including situations in which it is important to understand the location of the probe. Probes can be used during surgical procedures, such as cardiac surgical procedures in which a surgeon tracks a position of the probe relative to the anatomy of the heart. A surgeon typically must deflect or flex a tip of the probe during a procedure by using some type of actuator, such as a knob or piston, integrated within a handle of the probe. Due to the sensitive nature of the electronics within the probe, this displacement of the distal tip of the probe causes interference or noise, which is undesirable and makes it difficult to obtain accurate readings from electrodes within the distal tip of the probe.

Accordingly, it would be desirable to provide a solution that addresses noise or interference related issues associated with deflection of the probe's distal tip.

US2020/170525A1 describes A method includes receiving (i) a plurality of electrocardiogram (ECG) signals acquired by a mapping catheter at a plurality of locations on a surface of a heart of a patient, (ii) a reference ECG signal from a reference catheter positioned at a nominal location in a coronary sinus (CS) of the patient, and (iii) position signals indicative of a position of the reference catheter in the CS. US2010/076454A1 describes a positioning device for positioning an object on a surface. US2011/152712A1 describes impedance tissue identification in blood vessels.

### SUMMARY

The invention is as defined in claim 1. Certain surgical methods are described with reference to the assembly of the present invention. Whilst no claim is directed to these methods per se, the assembly is capable of being used and is intended to be used in such methods.

Multiple different aspects and components of the method and system are described herein.

### BRIEF DESCRIPTION OF THE DRAWINGS

A more detailed understanding can be had from the following description, given by way of example in conjunction with the accompanying drawings wherein:
Figure 1 illustrates an exemplary system including a probe.
Figure 2 illustrates a distal tip of the probe of Figure 1 according to one aspect.
Figure 3 illustrates a schematic view of the probe of Figure 1 according to one aspect.
Figure 4 illustrates a cross-sectional view of the probe of Figure 1 according to one aspect.
Figures 5A-5C illustrates various states of a sensor implemented within the probe of Figure 1.
Figure 6 is a cross-sectional view of the probe of Figure 1 according to another aspect.
Figure 7 is a schematic view of a probe including a sensor according to another aspect.
Figure 8 is a cross-sectional view through line VIII-VIII of the probe from Figure 7.
Figures 9A and 9B illustrate voltage measurements during raw deflection sensing and post filtering.
Figure 10A illustrates a process according to one aspect of this disclosure.
Figure 10B illustrates signals generated by at least one step shown in Figure 10A.
Figure 10C illustrates signals generated by at least one step shown in Figure 10A.
Figure 10D illustrates signals generated by at least one step shown in Figure 10A.

### DETAILED DESCRIPTION

As disclosed herein, systems, apparatuses and methods are provided that address issues related to static friction, noise, and other types of interference associated with engaging the actuator in a probe to displace a distal tip of the probe.

The term probe is used interchangeably with the term catheter herein, and one skilled in the art would understand that any type of sensing device could be implemented with the configurations disclosed herein.

As used herein, the term noise is generally used to refer to any unwanted disturbance of a signal. Noise can generally cause errors or undesired random disruptions in electrical signals.

In one aspect, the disclosed subject matter provides an arrangement in which deflection tracking is implemented within a probe assembly. Based on this tracking, any noise or interference can be identified and then filtered out of the signals generated by electrodes in the probe assembly.

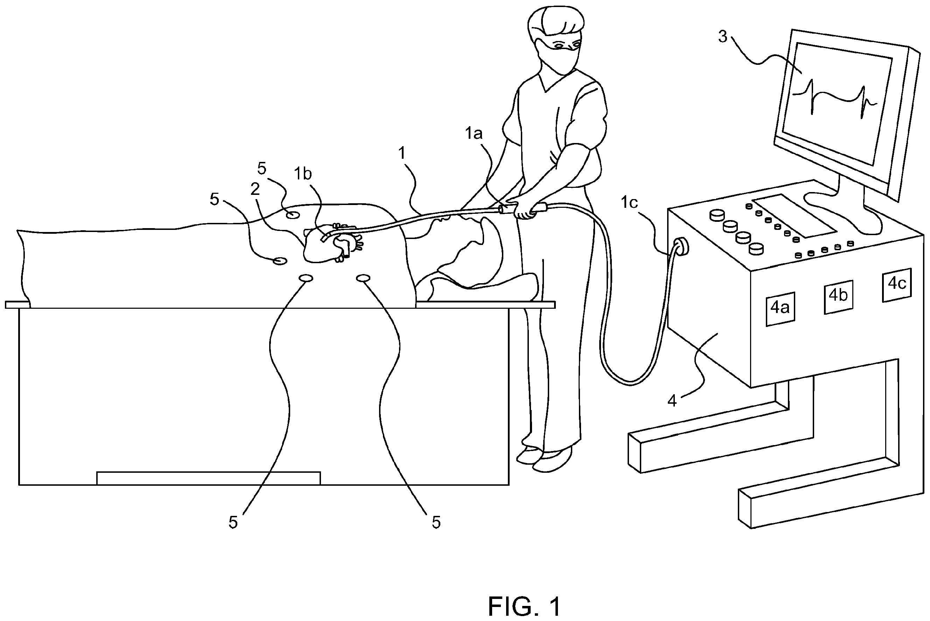

Figure 1 illustrates one embodiment for implementing aspects of the disclosed subject matter. As shown in Figure 1, a surgeon is navigating a probe 1 relative to a patient. In one embodiment, the surgeon is navigating the probe 1 within a patient's heart 2.

In one aspect, at least one sensor 5 is attached directly to the patient's body. In one embodiment, the sensor 5 is a patch that is configured to detect magnetic and/or electrical signals. In one embodiment, the sensors 5 are configured to measure impedance among the sensors 5. In another aspect, at least one single-axis magnetic sensor mounted on the catheter tip is configured to work in conjunction with at least one external magnetic sensor in a patient pad (i.e. under the patient). In one embodiment, there are three magnetic sensors mounted on the catheter that are oriented in three different directions (i.e. spaced 120 degrees apart) and that are configured to work in conjunction with three external magnetic sensors. Details of such technique are provided in the following documents: US Patent 5,391,199, US Patent 5,443,489, US Patent 5,558,091, US Patent 6,172,499, US Patent 6,177,792, US Patent 6,690,963, US Patent 6,788,967, and US Patent 6,892,091. In another aspect, an impedance sensor on the catheter is provided that is configured to be used without any external sensors. Details of such technique are provided in the following documents: US Patent 5,944,022, US Patent 5,983,126, and US Patent 6,456,864. These sensors generally assist with modeling a patient's respiratory cycle, identifying when a patient's lungs are breathing in or out, and tracking a location of the probe.

One skilled in the art would understand based on the present disclosure that the embodiments disclosed herein are not limited to a heart and can be implemented to analyze any type of body part or organ. On a monitor 3, the surgeon views various data sets and models related to respiration, probe motion and location, and probe-heart motion and location. The monitor 3 can be configured to display data regarding signals detected by the probe and the sensor.

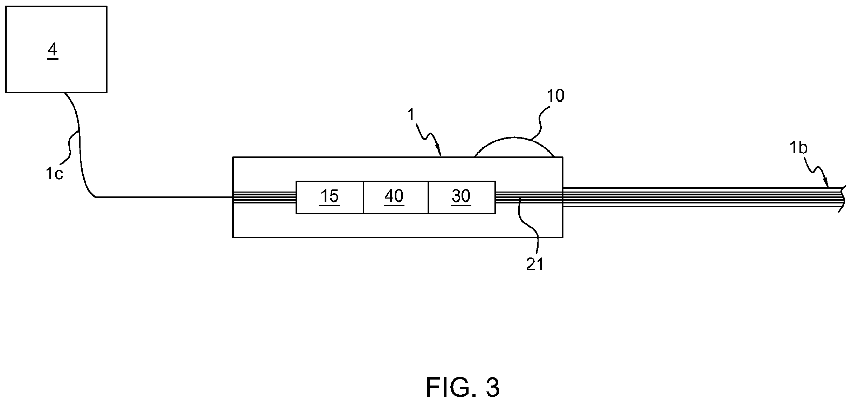

The probe 1 is also referred to as a probe assembly herein. The probe 1 can include a handle 1a, a distal tip 1b, and a proximal portion 1c connected to a computing system 4. The handle 1a includes an actuator 10, which is shown more clearly in Figures 3, 4, 6, and 7, configured to be engaged by a surgeon in order to manipulate elements at the distal tip 1b of the probe 1.

The computing system 4 is configured to implement various processes and algorithms disclosed herein. The computing system 4 can include a control unit 4a, a processor 4b, and a memory unit 4c. The control unit 4a can be configured to analyze signals from the probe 1 and sensors to determine coordinates and positions of the probe 1 as well as various other information. The memory unit 4c can be of various types, and is generally configured to track position data, respiration data, time data, and other types of data regarding the probe 1 and sensors. The computing system 4 can be configured to implement any of the steps, processes, methods, configurations, features, etc., that are disclosed herein.

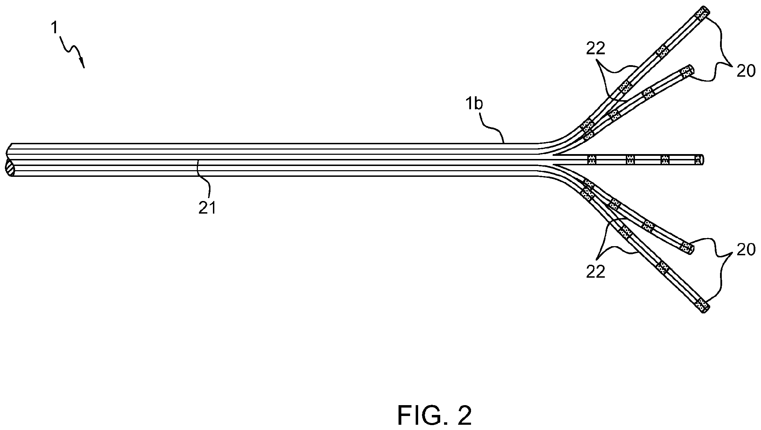

As shown in Figure 2, the distal tip 1b of the probe 1 includes a plurality of electrodes 20 arranged along a plurality of arms 22 of the probe. These electrodes 20 are electrically connected to the computing system 4 via electrical wires 21 (shown in Figures 2 and 3) within the arms 22. The electrodes 20 are configured to detect electrical signals in a patient, and more specifically can be configured to detect electrical signals generated by a patient's tissue when the electrodes 20 contact the patient's tissue. One skilled in the art would understand based on the present disclosure that the configuration of the electrodes 20 can vary. For example, although five arms 22 with electrodes 20 are illustrated in Figure 2, one skilled in the art would recognize that the exact shape and configuration of the distal tip 1b of the probe 1 can vary to include any number of arms 22, or to be arranged in a basket or balloon shape or other shape or form.

Figure 3 illustrates a schematic diagram of the probe 1 to show a sensor 30 integrated within the probe 1, and an actuator 10. When the actuator 10 is engaged or displaced, its motion is imparted onto the distal tip 1b of the probe 1. For example, engaging the actuator 10 can flex or deflect the arms 22 against various portions of the cardiac anatomy. The process of imparting this motion inherently causes noise or interference due to the wires 21 within probe 1 being placed under tension or otherwise being manipulated. This noise or interference makes it difficult to identify the signals from the electrodes 20.

As shown in Figure 3, the probe 1 (including the sensor 30) is connected to the computing system 4 and its associated components. All signals generated by the electrodes 20 in the tip 1b of the probe 1 and the sensor 30 are processed by the computing system 4.

The sensor 30 can be connected directly to the computing system 4, or to a printed circuit board or electrical circuitry 15 provided in the probe 1 between the sensor 30 and the computing system 4, or can be configured to transmit signals wirelessly. One skilled in the art would understand that the specific configuration of the electrical components of the probe 1 can vary.

The actuator 10 is shown generically in Figure 3. One skilled in the art would understand that exact form of the actuator 10 can vary, and could include a dial, knob, slider, or any other component configured to translate a surgeon's physical manipulation applied to the actuator 10 into corresponding movement imparted onto the distal tip 1b (i.e. the arms 22) of the probe 1. A surgeon can apply linear, rotational, or twisting manipulation to the actuator 10 such that the arms 22 of the probe 1 are flexed or deflected or otherwise displaced. The form of the sensor 30 can be adapted to be implemented into the various types of actuators 10. For example, if the actuator 10 is a knob or dial, the sensor 30 can include a marking on the knob or dial and an optical sensing device that is configured to detect the marking. Similarly, a magnetic target component can be arranged on the knob or dial, and a magnetic sensing component can be configured to detect the position of the magnetic target component.

In one aspect, the sensor 30 is configured to detect at least one of: (i) a position of the actuator 10, or (ii) noise generated by at least one wire 21 connected to an electrode 20 in the distal tip 1b of the probe 1. In a general aspect, the sensor 30 is configured to detect interference or noise experienced by the wires 21 connected to the electrodes 20. This configuration can be implemented in a variety of ways, such as by tracking the position or movement of the actuator 10 or by tracking impulses, tension, displacement, noise, or electrostatic experienced directly by the wires 21 connected to the electrodes 20. One skilled in the art would recognize based on the present disclosure that other configurations can be implemented to identify the noise or interference experienced by the wires 21.

The sensor 30 disclosed herein can be implemented in a variety of ways. For example, the sensor 30 can be implemented as: a capacitive displacement sensor, a sliding resistor displacement sensor, an optical encoder, a Hall-effect sensor, piezoelectric sensor, or any other type of sensor. In one embodiment, the sensor 30 consists of electrical sensing wires, which are described in more detail herein.

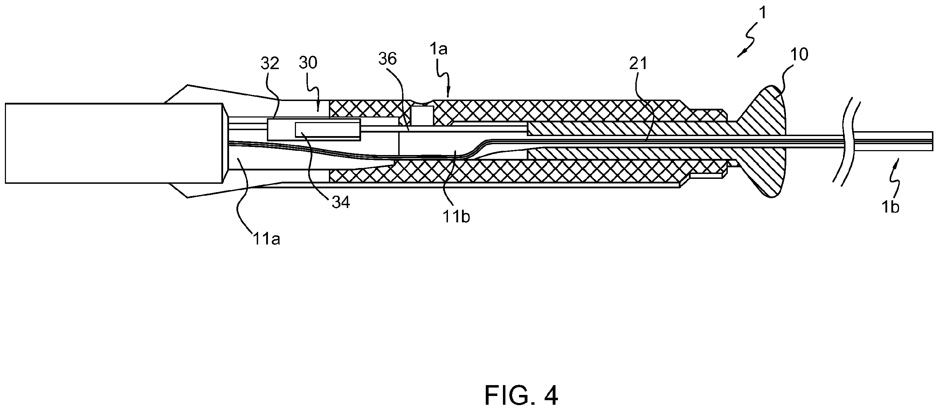

Figure 4 illustrates a more detailed view of the handle 1a of the probe 1. As oriented in Figure 4, the distal tip 1b of the probe 1 is on the right-hand side of the cross-section. The actuator 10 is shown as a piston in Figure 4. Figure 4 illustrates one exemplary configuration for implementing the sensor 30 within the handle 1a of the probe 1. As shown in Figure 4, the sensor 30 includes a stationary component 32 and a mobile component 34. In one aspect, the mobile component 34 is connected to the actuator 10. As shown in Figure 4, a linkage 36 is provided that connects the actuator 10 to the sensor 30. The mobile component 34 can be directly connected to the actuator 10 in one embodiment. In another embodiment, intermediate connection elements can be provided to provide the linkage between the mobile component 34 and the actuator 10.

The handle 1a of the probe 1 can include various chambers or cavities. As shown in Figure 4, the sensor 30 is implemented within a cavity 11a. In other embodiments, it is understood by those of skill in the art that the sensor 30 can be implemented in another cavity 11b, or any other region or portion of the probe 1. In other embodiments, the sensor 30 is implemented in regions of the probe 1 other than the handle 1a.

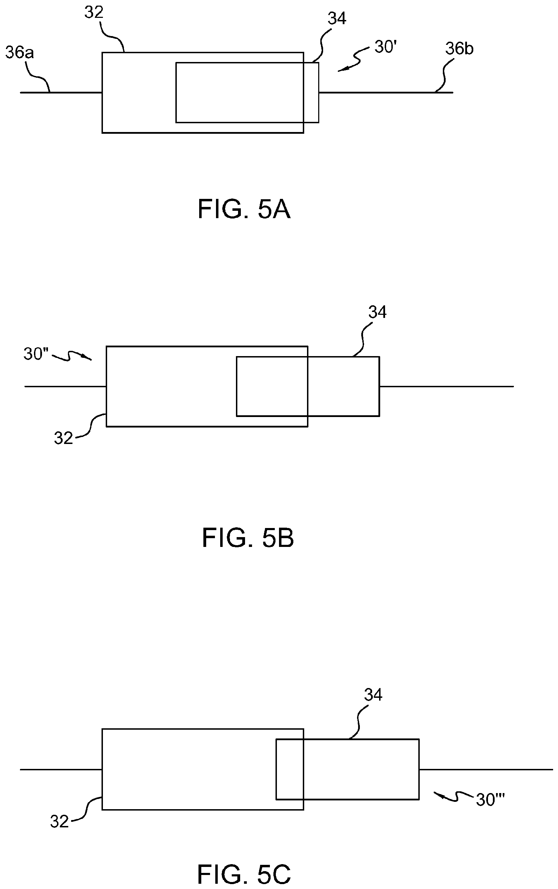

Figures 5A-5C illustrates the sensor 30 in varying states 30', 30", and 30‴. In each of these Figures, a mobile component 34 is slidably received inside of a bore defined by the stationary component 32. In one embodiment, the stationary component 32 and the mobile component 34 are cylindrical components. One skilled in the art would understand that the shape of these components can vary. As shown in Figure 5A, the linkage 36 includes a first portion 36a that connects the stationary component 32 to a body of the probe 1. The linkage 36 also includes a second portion 36b that connects the mobile component 34 to the actuator 10. The first and second portions 36a, 36b are shown schematically in Figures 5A-5C, but one skilled in the art would understand that these components can be modified. For example, the stationary component 32 can be press fit inside a cavity defined by the handle 1a of the probe 1. In another example, the second portion 36b can be omitted and the mobile component 34 can be directly attached to the actuator 10.

As shown in Figure 5A, the mobile component 34 is almost entirely inside of the stationary component 32. This state corresponds to a first capacitance value C1. Figure 5B corresponds to a state in which about a half of the mobile component 34 is inside of the stationary component 32. Figure 5B corresponds to a state having a second capacitance value C2. Figure 5C corresponds to another state in which the mobile component 34 is almost entirely outside of the stationary component 32. A third capacitance value C3 is provided by the arrangement in Figure 5C. Based on the varying capacitance values C1, C2, and C3, it is possible to determine a position of the actuator. In one embodiment, C1 > C2 > C3. One skilled in the art would understand that the arrangements shown in Figures 5A-5C can be modified.

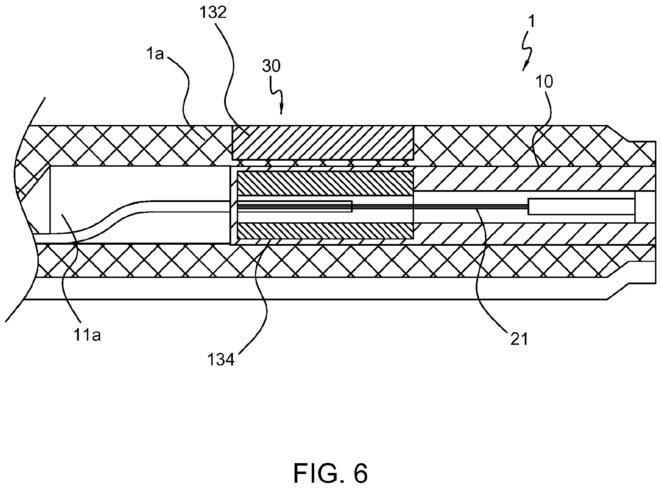

Figure 6 illustrates another arrangement for a sensor 30. In this configuration, the sensor 30 is provided in cavity 11b and in more direct contact with the actuator 10. In this arrangement, the sensor 30 includes a stationary component or plate 132, and a mobile component 134 attached to the actuator 10. In this embodiment, the stationary component 132 can consist of a plate, and more specifically can consist of a copper plate. Similarly, the mobile component 134 can also consist of a copper plate. Various types of materials can be used to form the components 132, 134, as one skilled in the art would recognize based on the present disclosure. These components 132, 34 are connected to an electrical circuit 15 or the computing system 4.

In one embodiment, the sensor 30 is implemented as force sensing linear potentiometer. As the actuator 10 is displaced due to manual manipulation, the actuator 10 can engage a strip or pad configured to deflect or otherwise be manipulated by the actuator 10. As the actuator 10 moves, the sensor 30 can detect a position of the actuator 10, which is then transmitted to the computing system 4. In other words, the sensor 30 detects a relative position of the actuator 10 and then an output signal, such as a resistance value, is provided to indicate a position of the actuator 10.

In another embodiment, the sensor 30 is implemented as a conductive film sensor assembly. For example, a semi-conductive material layer can be applied to the actuator 10. The semi-conductive material layer can be shrink wrapped around the actuator 10, and a corresponding sensor 30 can be arranged to detect a position of the actuator 10.

As disclosed in the various embodiments, a sensor 30 is provided that generally detect a position of the actuator 10. Based on the position of the actuator 10, the computing system 4 is configured to determine a velocity of the actuator 10. In general, the greater the speed or velocity of the actuator 10, then the greater the resulting noise or interference experienced by the electrode wires 21.

In any one of the arrangements disclosed herein, the sensor 30 can consist of an accelerometer, or can include a secondary sensor in the form of an accelerometer. As shown in Figure 3, an accelerometer 40 is provided in the probe 1 and is configured to detect motion of the actuator 10. One skilled in the art would understand that additional sensing components can be implemented into the probe 1 to track motion of the actuator 10 or movement of the electrode wires 21.

In any one of the configurations disclosed herein, the sensor 30 provides information regarding displacement of the actuator 10 of the probe 1 that controls deflections or flexing of the electrodes 20 provided at the distal tip 1b of the probe 1. This information can then be used by the computing system 4 in order to determine a velocity of the actuator 10. Using this information, the computing system 4 can then identify time periods during which the actuator 10 is being displaced above a threshold velocity. If the actuator 10 is moving above a predetermined threshold velocity, then the computing system 4 can filter out signals or data obtained during those time intervals because the data or information obtained during those time intervals have a high likelihood of suffering from noise or interference. In one aspect, a relatively fast threshold velocity is 10 cm per second, and a relatively slow threshold velocity is 0.5 - 1.0 cm per second. Signals detected during the fast movement can be blanked out or further processed. One skilled in the art would understand that these values can vary depending on catheter design and translational movement, as well as tension. This noise or interference makes it difficult for surgeons to ascertain accurate electrophysiological signals being detected by electrodes 20 in the distal tip 1b of the probe 1. In other aspects, the sensor 30 is configured to detect acceleration of the actuator 10.

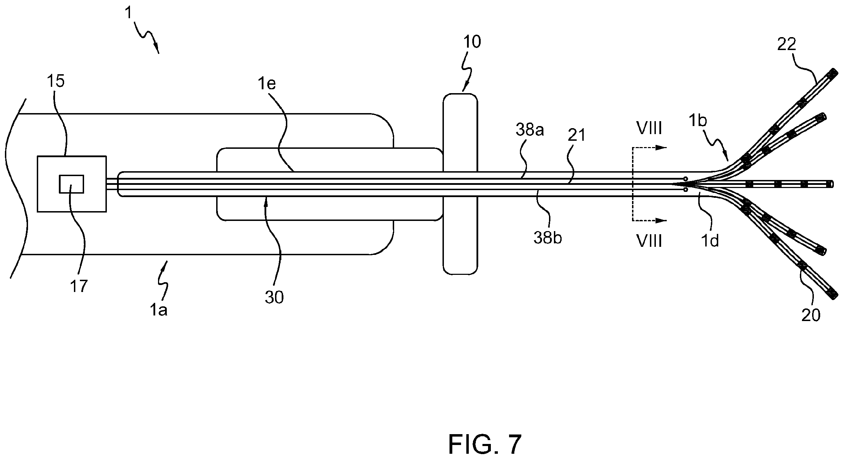

Figure 7 illustrates another aspect for identifying an impact of the actuator 10 deflecting or flexing the distal tip 1b of the probe 1. Figure 7 illustrates a simplified schematic view of a probe 1 in which the sensor 30 consists of two sensing wires 38a, 38b. These sensing wires 38a, 38b are positioned adjacent to the wires 21 that are connected to the electrodes in the distal tip 1b of the probe 1. In one aspect, the sensing wires 38a, 38b act as leads or antennae. In other words, the sensing wires 38a, 38b are configured to detect voltage fluctuations or other changes experienced by the electrode wires 21.

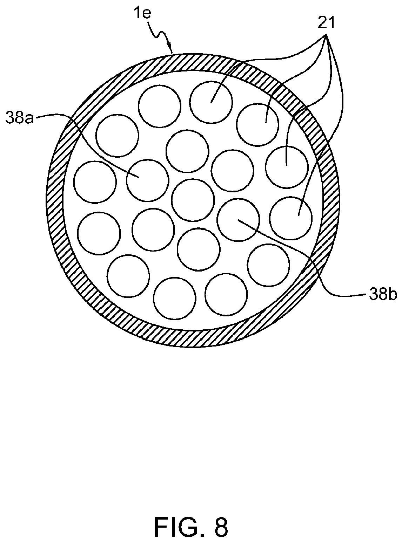

A cable sheath 1e is shown in Figure 7, and the cable sheath 1e is shown in more detail in Figure 8. Inside the cable sheath 1e, a plurality of electrode wires 21 as well as the sensing wires 38a, 38b are bundled together. All of the remaining wires inside of the sheath 1e not specifically annotated in Figure 8 are electrode wires 21. In one aspect, the wires 21, 38a, 38b are packed tightly together in a bundle such that any tension or motion experienced by the electrode wire 21 is also experienced by the sensing wires 38a, 38b. One skilled in the art would understand that the wires 21, 38a, 38b do not need to be bundled together and other arrangements are possible in which the sensing wires 38a, 38b are in close proximity or otherwise engaged with the electrode wire 21. Additionally, the bundle of wires in Figure 8 are shown in a specific shape and arrangement, however one skilled in the art would understand that the shape and arrangement can be modified.

All of the wires 21, 38a, 38b are commonly connected to electrical circuitry 15, which is shown schematically in Figure 7. The electrical circuitry 15 is connected to the computing system 4. The electrical circuitry 15 can be connected to any other component and can be generally configured to receive, process, and/or send signals to and from the wires 21, 38a, 38b. A resistor, shown schematically as element 17 in Figure 7, can be implemented between the sensing wires 38a, 38b in electrical circuitry 15. In one aspect, the resistor is positioned within the handle of the probe 1. In another embodiment, the resistor is positioned within one of the wires 38a, 38b. The electrical circuitry 15 can include any electrical components, such as resistors, conductors, capacitors, voltage sources, etc., and the electrical circuitry 15 can be arranged in any configuration such that loads, impulses, forces, tension, or any other signals from the wires 21, 38a, 38b are detected.

The sensing wires 38a, 38b are not connected to any of the electrodes 20 defined by the distal tip 1b of the probe 1 and are isolated from the electrodes 20. Instead, the sensing wires 38a, 38b terminate at some area (such as area 1d in Figure 6) short of the electrodes 20 and the tips of the arms 22. In one embodiment, two sensing wires 38a, 38b are provided. One skilled in the art would understand that a single sensing wire 38a or more than two sensing wires 38a, 38b can be used. In another configuration, a single sensing wire can be implemented that is configured to measure potential relative to some ground.

For illustrative purposes and to simplify the drawing, only one electrode wire 21 is shown in Figure 7 however one skilled in the art would understand that a plurality of sensing wires 21 are provided. In one embodiment, each electrode 20 in the distal tip 1b of the probe 1 is connected to a respective electrode wire 21 and therefore the number of electrode wires 21 can vary greatly depending on the configuration of the distal tip 1b of the probe 1.

The sensor 30 formed by the sensing wires 38a, 38b is configured to track and identify noise caused by friction, and more specifically cause by electrostatic friction associated with the electrode wires 21. The sensing wires 38a, 38b can be provided in any region of the probe 1, including the handle 1a, distal tip 1b (short of the electrodes 20), or proximal portion 1c. Because the sensing wires 38a, 38b do not connect to electrodes, the sensing wires 38a, 38b do not generate any local signal measurements regarding a patient's tissue. Instead, the sensing wires 38a, 38b are specifically configured to be affected by noise generated by the electrode wires 21. As electrostatic is generated by movement of the electrode wires 21 during deflection, then a potential of the sensing wires 38a, 38b is modified, causing a voltage change that can be measured and used to detect the presence of electrostatic discharge. In this aspect, the sensing wires 38a, 38b therefore function and operate as a sensor.

In one aspect, the computing system 4 is configured to receive signals from the sensor 30 (regardless of how it is implemented or embodied, i.e. as a displacement sensor, sensing wires, or any other configuration), and to identify noise or interference due to the electrode wires 21 being under tension, moved, or otherwise impacted while the actuator 10 is engaged.

The computing system 4 can be configured to filter or blank out intervals of signals generated by the electrode wires 21 during periods when the noise is above a predetermined noise threshold. In other words, the computing system 4 is configured to identify specific episodes during which there is an unacceptable level of noise and can automatically filter out those episodes. The quantity of noise can vary based on circuit design, as one skilled in the art would understand based on the present application.

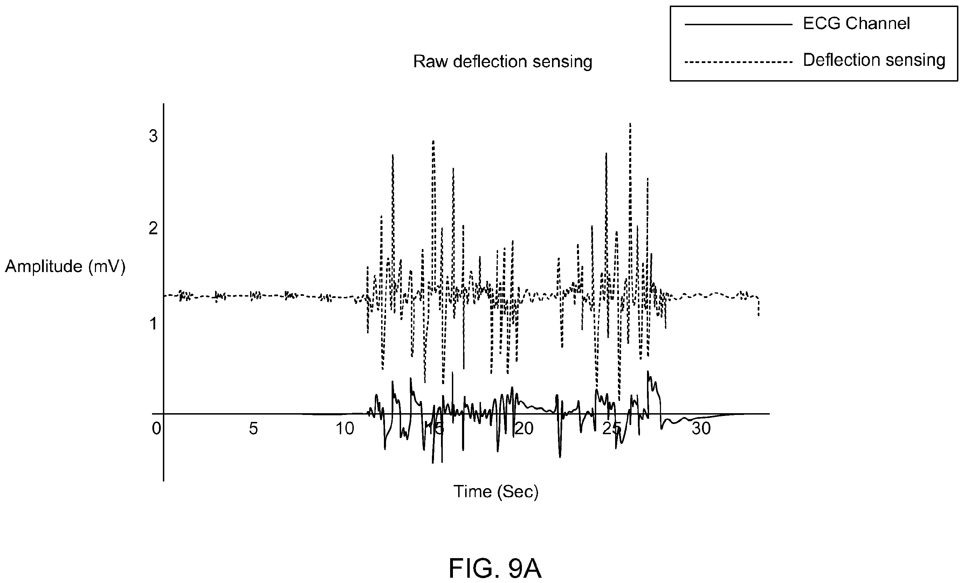

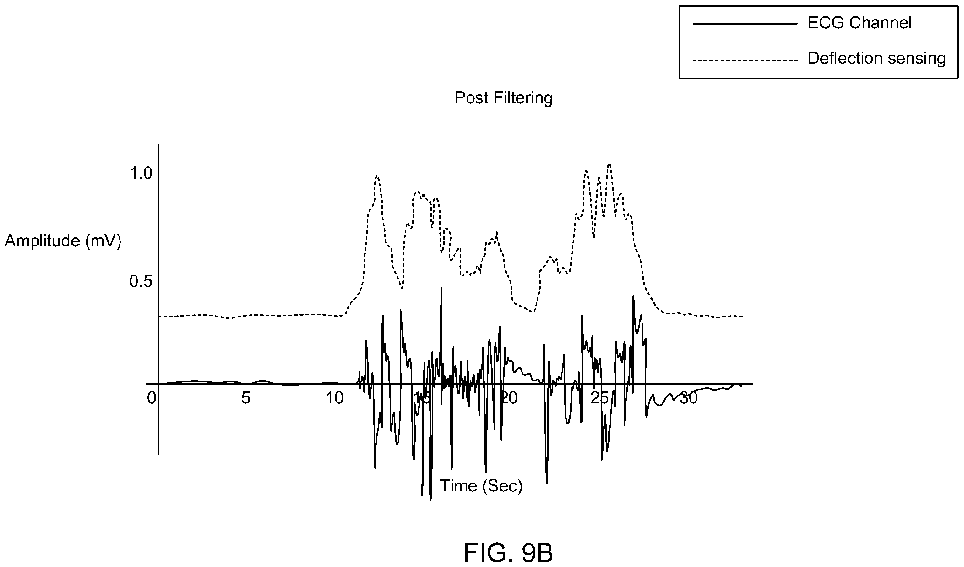

In one aspect, if a resistor having a relatively lower resistance (i.e. 1 Ω) is used, then the resulting detected voltage would generally also be low. In another aspect, if a resistor having a relatively higher resistance (i.e. 10 M Ω) is used, then the resistor will detect much greater noise from the power outlet. Accordingly, a resistor having relatively moderate resistance (i.e. 5 KΩ - 50 KΩ) is generally preferable in one aspect. A baseline level of noise can be established by using the probe 1 in a clinical setup or setting in order to essentially calibrate the sensing configuration. This process involves identifying events of noise by intentionally generating noise, either by manual manipulation or exceeding speed thresholds of movement. Then, the noise measured by the sensing wires 38a, 38b can be compared and specific noise events can be analyzed to establish a baseline or cutoff threshold during which particularly high noise episodes can be rejected or blanked out. The data associated with these sensing steps and post filtering are shown in Figures 9A and 9B. Figures 9A and 9B represent testing data, in which one electrode is connected to a resistor with 30 KΩ. Figure 9A illustrates signals as recorded from the two sensing wires 38a, 38b. The dashed lines correspond to deflection sensing from the sensing wires 38a, 38b, and the solid lines illustrates the electrocardiograph (ECG) signal from an electrode. The middle region of the chart in which the lines both show the most activity corresponds to a period when the ECG signal has energy (i.e. deflection noise). During this energized state, the dashed line which represents the signals from the sensing wires 38a, 38b also shows activity. Figure 9B is provided to illustrate how the dashed line (corresponding to the signals of the sensing wires 38a, 38b) is filtered in order to identify regions of deflection noise energy (dashed line).

Further processing steps can be performed by algorithms, processes, or other functions programmed into the computing system 4. In one aspect, addressing the unwanted noise detected by the sensors can further include rectifying the detected signal using processing, such as via a root mean square (RMS) function. The detected signal can be filtered using high pass and low pass filters. High pass filtering can remove localized components. In other words, when the signal is "floating" (i.e. not on the zero line), the baseline can be removed. Low pass filtering smooths the signals by removing high frequency components, which is shown as the "deflection sensing" data in Figures 9A and 9B.

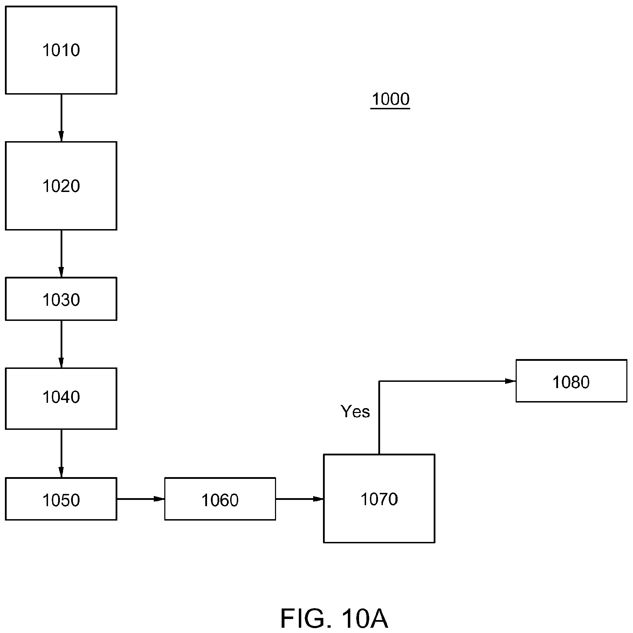

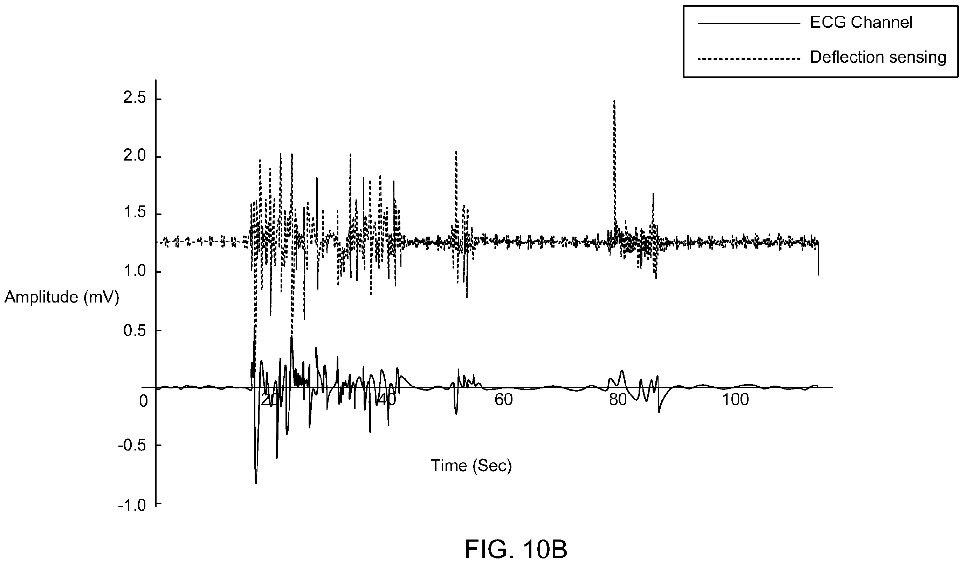

A flow chart is illustrated in Figure 10A that generally illustrates a process 1000. Figures 10B-10D illustrate an ECG signal from the electrode (solid line) and/or the sensor (dashed lines).

As shown in Figure 10A, step 1010 includes sampling a signal from a sensor (i.e. sensor 30). In one aspect, the sensor includes two sensing wires (i.e. wires 38a, 38b). One skilled in the art would understand that the sensor can include any of the other configurations disclosed herein. The data from step 1010 is represented in Figure 10B.

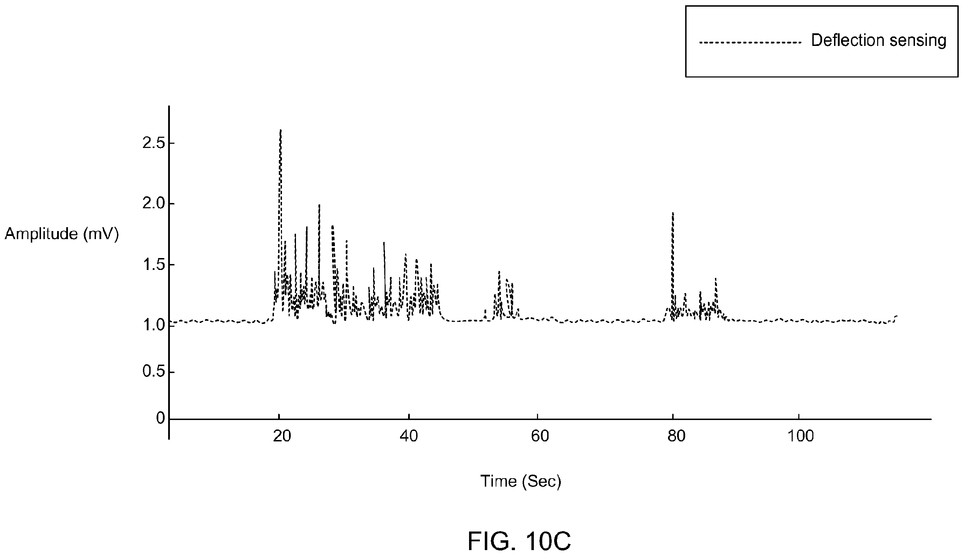

Step 1020 includes a filtering step. In one aspect, the filtering step includes filtering power line noise. The filter can be a comb filter type that is configured to attenuate energy at 50 Hz and/or 60 Hz and the associated harmonics. This step is configured and calibrated to be sensitive to deflection. Step 1030 applies an absolute filter to the signals from step 1020. Step 1040 includes applying additional filters, such as a high pass (i.e. 0.5 Hz) and/or a localization filter which subtracts a local mean signal or removes the baseline. After steps 1020, 1030, and 1040, Figure 10C illustrates the remaining signal. Figure 10C illustrates an amplitude baseline of 1mV, but one skilled in the art would understand that the baseline could be closer to 0 mV.

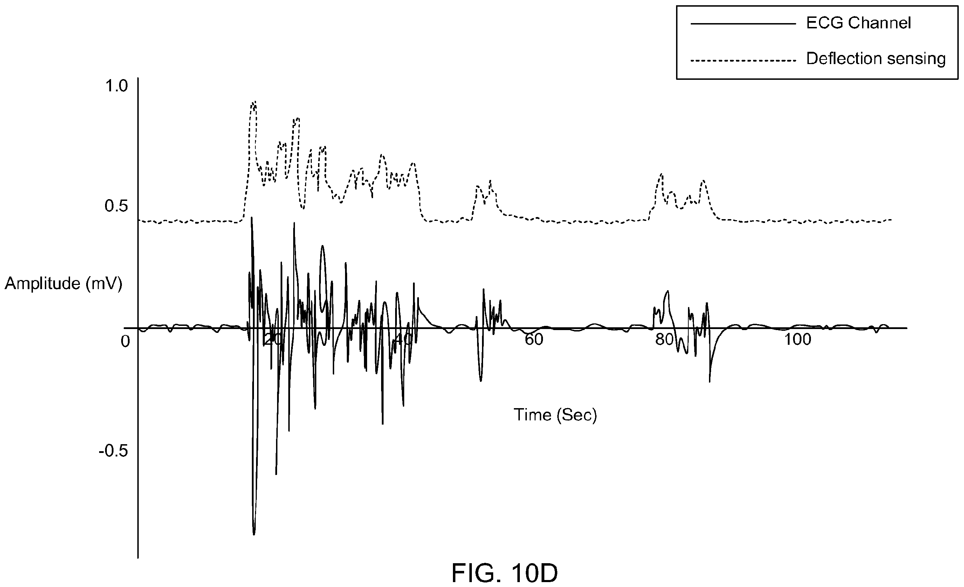

Step 1050 includes applying a low pass filter having a predetermined setting, such as a one-second running average. This process essentially smooths out small spikes and presents an average energy value, as shown by the signals in Figure 10D. The sensor signal (shown with dashed lines) is low in the intermediate periods when there is no energy in the ECG signal (solid line).

Step 1060 includes setting a threshold value for noise. One skilled in the art would understand that step 1060 can be performed prior to any one or more of the steps described herein. Step 1070 includes checking whether the detected signals are above the threshold. If it is determined that the detected signals are above the threshold in step 1070, then step 1080 includes detecting deflection. In one embodiment, an alert can be triggered when the threshold signal detected by the sensor is exceeded for a predetermined period (i.e. 100 ms). One skilled in the art would understand that differing alerting systems or monitoring systems can be implemented using the concepts disclosed herein.

In one aspect, the disclosed subject matter does not merely apply a filter to raw signals from the electrode wires 21 and instead uses additional information or signals collected by the sensor 30 to analyze the electrode wire signals to account for the noise caused by the actuator 10 and its deflection of the electrode wires 21.

In one aspect, when the sensor 30 is configured to detect displacement and velocity of the actuator 10, the computing system 4 can be configured to specifically blank out or filter out signals generated during intervals when the actuator 10 is moving above a predetermined velocity threshold. The computing system 4 can also be configured to apply high or low pass filters or smoothing filters or functions to the signals received by the electrodes 20 and the sensor 30. In one aspect, the signals detected by the sensor 30 can be provided to a surgeon or physician via the monitor 3 or other display means and no filtering or blanking is required.

The subject matter disclosed herein addresses issues caused by deflecting or flexing the distal tip 1b of the probe 1, which inherently causes the electrode wires 21 to be affected. By monitoring the actuator 10 or the electrode wires 21, it is possible to pinpoint moments and signals that are impacted by the deflection or flexing of the distal tip 1b of the probe 1 according to the disclosed subject matter.

The subject matter disclosed herein can be implemented using any one or more of the following Biosense Webster, Inc. components or interfaces: CARTO^{®} 3 System Qmode+ Software, Qdot Catheter, nMARQ^{™} RF Generator and Coolfow Pump, VISITAG^{®} module, and Pentaray Nav Catheter. One skilled in the art would understand that the disclosed subject matter could be implemented with various other components and interfaces.

The disclosed subject matter is not limited to being used in connection with a human patient, or a patient's heart. The disclosed subject matter can be used in a variety of applications to analyze features of any type of object, such as a chamber. Additionally, the sensing configuration can be used in non-medical applications.

Any of the functions and methods described herein can be implemented in a general-purpose computer, a processor, or a processor core. Suitable processors include, for example, a general-purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine.

Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general-purpose computer or a processor.

Examples of non-transitory computer-readable storage mediums include a read only memory (ROM), a random-access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

## Claims

1. A probe assembly for use during a surgical procedure, the probe assembly comprising:
a probe (1) including a distal tip (1b) with a plurality of electrodes (20) connected to a plurality of electrode wires (21), and an actuator (10) configured to displace the distal tip;
a sensor (30) configured to detect at least one of:
(i) a position of the actuator during displacement of the distal tip, or
(ii) electrical noise generated by the plurality of electrode wires during displacement of the distal tip; and
a processor (4b), **characterized in that** the processor is configured to:
(i) determine a velocity of the actuator based on the position of the actuator, identify intervals during which the velocity of the actuator is above a predetermined velocity threshold, and blank out signals or filter signals that are obtained during periods when a velocity of the actuator is above a predetermined velocity threshold, or
(ii) blank out signals or filter signals that are obtained during periods when the noise generated by the plurality of electrode wires during displacement of the distal tip is above a predetermined noise threshold.

2. The probe assembly of claim 1, wherein the sensor comprises at least one sensing wire (38a, 38b) configured to detect the noise generated by the plurality of electrode wires.

3. The probe assembly of claim 2, wherein the at least one sensing wire is positioned within a handle body (1a) of the probe assembly.

4. The probe assembly of claim 2, wherein the at least one sensing wire is positioned within a probe cable assembly bundle that also includes the plurality of electrode wires, the at least one sensing wire terminates before the distal tip, and the at least one sensing wire and the plurality of electrode wires are connected to a common electrical circuit (15).

5. The probe assembly of claim 2, wherein the at least one sensing wire is isolated from the plurality of electrodes.

6. The probe assembly of claim 1, wherein the sensor includes a first sensor component (34) attached to the actuator and a second sensor component (32) attached to a body of the probe, such that the first sensor component is mobile relative to the body of the probe and the second sensor component is stationary relative to the body of the probe.

7. The probe assembly of claim 1, wherein the sensor comprises at least one of: a capacitive displacement sensor, an accelerometer (40), a Hall-effect sensor, an optical sensor, a resistor displacement sensor, or a magnetic sensor.

## Patentansprüche

1. Sondenanordnung zur Verwendung während einer chirurgischen Prozedur, wobei die Sondenanordnung umfasst:
eine Sonde (1), aufweisend eine distale Spitze (1b) mit mehreren Elektroden (20), die mit mehreren Elektrodendrähten (21) verbunden sind, und einem Aktuator (10), der dafür ausgelegt ist, die distale Spitze zu verschieben;
einen Sensor (30), der dafür ausgelegt ist, wenigstens eines hiervon zu detektieren:
(i) eine Position des Aktuators während der Verschiebung der distalen Spitze oder
(ii) elektrisches Rauschen, das von den mehreren Elektrodendrähten während der Verschiebung der distalen Spitze erzeugt wird; und
einen Prozessor (4b), **dadurch gekennzeichnet, dass** der Prozessor ausgelegt ist zum:
(i) Bestimmen einer Geschwindigkeit des Aktuators, basierend auf der Position des Aktuators, Identifizieren von Intervallen, in denen die Geschwindigkeit des Aktuators über einem vorbestimmten Geschwindigkeitsschwellwert liegt, und Ausblenden von Signalen oder Filtern von Signalen, die in Zeiträumen erhalten werden, in denen eine Geschwindigkeit des Aktuators über einem vorbestimmten Geschwindigkeitsschwellwert liegt, oder
(ii) Ausblenden von Signalen oder Filtern von Signalen, die in Zeiträumen erhalten werden, in denen das von den mehreren Elektrodendrähten während einer Verschiebung der distalen Spitze erzeugte Rauschen über einem vorbestimmten Rauschschwellwert liegt.

2. Sondenanordnung nach Anspruch 1, wobei der Sensor wenigstens einen Sensordraht (38a, 38b) umfasst, der dafür ausgelegt ist, das von den mehreren Elektrodendrähten erzeugte Rauschen zu detektieren.

3. Sondenanordnung nach Anspruch 2, wobei der wenigstens eine Sensordraht in einem Griffkörper (1a) der Sondenanordnung positioniert ist.

4. Sondenanordnung nach Anspruch 2, wobei der wenigstens eine Sensordraht in einem Sondenkabelanordnungsbündel positioniert ist, das auch die mehreren Elektrodendrähte beinhaltet, der wenigstens eine Sensordraht vor der distalen Spitze endet und der wenigstens eine Sensordraht und die mehreren Elektrodendrähte mit einer gemeinsamen elektrischen Schaltung (15) verbunden sind.

5. Sondenanordnung nach Anspruch 2, wobei der wenigstens eine Sensordraht von den mehreren Elektroden isoliert ist.

6. Sondenanordnung nach Anspruch 1, wobei der Sensor eine erste Sensorkomponente (34) aufweist, die an dem Aktuator angebracht ist, und eine zweite Sensorkomponente (32), die an einem Körper der Sonde angebracht ist, sodass die erste Sensorkomponente relativ zu dem Körper der Sonde beweglich ist und die zweite Sensorkomponente relativ zu dem Körper der Sonde stationär ist.

7. Sondenanordnung nach Anspruch 1, wobei der Sensor wenigstens eines hiervon umfasst: einen kapazitiven Verschiebungssensor, einen Beschleunigungsmesser (40), einen Hall-Effekt-Sensor, einen optischen Sensor, einen Widerstandsverschiebungssensor oder einen Magnetsensor.

## Revendications

1. Ensemble formant sonde à utiliser au cours d'une procédure chirurgicale, l'ensemble formant sonde comprenant :
une sonde (1) comprenant une pointe distale (1b) avec une pluralité d'électrodes (20) connectées à une pluralité de fils d'électrodes (21), et un actionneur (10) configuré pour déplacer la pointe distale ;
un capteur (30) configuré pour détecter au moins l'un des éléments suivants :
(i) une position de l'actionneur pendant le déplacement de la pointe distale, ou
(ii) le bruit électrique généré par la pluralité de fils d'électrodes pendant le déplacement de la pointe distale ; et
un processeur (4b), **caractérisé par le fait que** le processeur est configuré pour :
(i) déterminer une vitesse de l'actionneur sur la base de la position de l'actionneur, identifier les intervalles pendant lesquels la vitesse de l'actionneur est supérieure à un seuil de vitesse prédéterminé, et supprimer les signaux ou filtrer les signaux qui sont obtenus pendant les périodes où la vitesse de l'actionneur est supérieure à un seuil de vitesse prédéterminé, ou
(ii) supprimer les signaux ou filtrer les signaux obtenus pendant les périodes où le bruit généré par la pluralité de fils d'électrodes pendant le déplacement de l'extrémité distale est supérieur à un seuil de bruit prédéterminé.

2. Ensemble formant sonde selon la revendication 1, dans lequel le capteur comprend au moins un fil de détection (38a, 38b) configuré pour détecter le bruit généré par la pluralité de fils d'électrodes.

3. Ensemble formant sonde selon la revendication 2, dans lequel l'au moins un fil de détection est positionné dans un corps de poignée (1a) de l'ensemble formant sonde.

4. Ensemble formant sonde selon la revendication 2, dans lequel l'au moins un fil de détection est positionné dans un faisceau de câbles de sonde qui comprend également la pluralité de fils d'électrodes, l'au moins un fil de détection se terminant avant la pointe distale, et l'au moins un fil de détection et la pluralité de fils d'électrodes étant connectés à un circuit électrique commun (15).

5. Ensemble formant sonde selon la revendication 2, dans lequel l'au moins un fil de détection est isolé de la pluralité d'électrodes.

6. Ensemble formant sonde selon la revendication 1, dans lequel le capteur comprend un premier composant de capteur (34) fixé à l'actionneur et un deuxième composant de capteur (32) fixé à un corps de la sonde, de sorte que le premier composant de capteur est mobile par rapport au corps de la sonde et que le deuxième composant de capteur est stationnaire par rapport au corps de la sonde.

7. Ensemble formant sonde selon la revendication 1, dans lequel le capteur comprend au moins l'un des éléments suivants : un capteur de déplacement capacitif, un accéléromètre (40), un capteur à effet Hall, un capteur optique, un capteur de déplacement à résistance ou un capteur magnétique.
